# EUROPÄISCHE PATENTSCHRIFT

(11) **EP 2 578 201 B1**
(45) Veröffentlichungstag und Bekanntmachung des Hinweises auf die Patenterteilung: **25.11.2020**
(21) Anmeldenummer: 11184237.3
(22) Anmeldetag: 06.10.2011
(51) Int. Cl.: A61K 6/00

(54) **Dentalmaterialien mit verbesserter Hydrolysestabilität auf der Basis von Phthalsäuremonomeren**
Dental materials with improved hydrolysis stability on the basis of phthalic acid monomers
Matériaux dentaires dotés d'une stabilité à l'hydrolyse améliorée à base de monomères d'acide phtalique

(43) Veröffentlichungstag der Anmeldung: 10.04.2013
(73) Patentinhaber: Ivoclar Vivadent AG, 9494 Schaan (LI)
(72) Erfinder: Bock, Thorsten, 6806 Tosters (AT); Fischer, Urs-Karl, 9320 Arbon (CH); Lamparth, Iris, 94723 Grabs (CH); Moszner, Norbert, 9493 Mauren (LI); Rheinberger, Volker, 9490 Vaduz (LI)
(74) Vertreter: Uexküll & Stolberg

(56) Entgegenhaltungen:
- JP-A- H02 251 507
- US-A- 4 521 550
- DATABASE WPI Week 201127 Thomson Scientific, London, GB; AN 2011-D06252 XP002670866, & JP 2011 057657 A (MATSUKAZE KK) 24. März 2011 (2011-03-24)
- DATABASE WPI Week 198232 Thomson Scientific, London, GB; AN 1982-66602E XP002670867, & JP 57 105409 A (LION CORP) 30. Juni 1982 (1982-06-30)
- DATABASE WPI Week 199320 Thomson Scientific, London, GB; AN 1993-164315 XP002670881, & JP 5 097621 A (MITSUI PETROCHEM IND CO LTD) 20. April 1993 (1993-04-20)

## Beschreibung

Die vorliegende Erfindung betrifft polymerisationsfähige Phthalsäuremonomere mit hoher Hydrolysestabilität und deren Verwendung als Monomerkomponente in adhäsiven Dentalmaterialien, insbesondere zur Herstellung von dentalen Adhäsiven und Zementen.

4-Methacryloyloxyethyltrimellitsäure (4-MET) und das entsprechende Anhydrid (4-META) zeigen als Haftmonomere in dentalen Adhäsiven ausgezeichnete Hafteigenschaften, sowohl auf der Zahnhartsubstanz als auch auf metallischen Substraten, und haben deshalb in vielen Dentinhaftvermittlern verschiedenster Generationen sowie in Metallprimern Anwendung gefunden (Chang et al., "4-META use in dentistry: a literature review", J. Prosthet. Dent. 87 (2002) 216-224). 4-META wird in wässrigen Lösungen sehr schnell zu 4-MET hydrolysiert, und es wird angenommen, dass 4-MET für die Haftungseigenschaften verantwortlich ist (S. Fujisawa, S. Ito, Dent. Mat. J. 18 (1999) 54-62).

Wässrige Lösungen von 4-MET sind aber nicht lagerstabil, da die Methacrylatgruppe hydrolytisch abgespalten wird. Es wurde gefunden, dass in wässrigen Lösung bereits nach achtwöchiger Lagerung bei 42 °C ein großer Teil der ursprüngliche eingesetzten Verbindung hydrolytisch abgebaut wird. Damit ist der Einsatz von 4-META und 4-MET in selbstätzenden Schmelz-Dentin-Adhäsiven, die Wasser als Co-Lösungsmittel enthalten, sehr problematisch.

Die EP 1 681 283 A1 offenbart Etherverbindungen organischer Säuren und Anhydride wie 4-(2-Ethoxycarbonyl-allyloxy)-phthalsäure, die hydrolysestabil sein und sich zur Herstellung selbstätzender Dentaladhäsive eignen sollen. Die Hydrolysestabilität dieser Verbindungen ist allerdings ebenfalls nicht zufriedenstellend.

Die WO 03/035013 A1 offenbart wässrige, einkomponentige, selbstätzende Dentaladhäsive mit einem pH-Wert von maximal 2, die ein polymerisierbares N-substituiertes Alkylacryl- oder Acrylamidmonomer enthalten, das mindestens eine Phosphonsäure- oder Sulfonsäuregruppe aufweist. Die Monomere sollen hydrolysestabil sein.

Der Erfindung liegt die Aufgabe zugrunde, adhäsive Dentalmaterialien auf der Basis von Phthalsäure-Derivaten bereitzustellen, die sehr gut polymerisationsfähig sind, unter sauren Bedingungen eine hohe Hydrolysestabilität zeigen, sich in polaren Lösungsmitteln lösen, eine gute Substrathaftung auf Zahnhartsubstanz vermitteln und die physiologisch unbedenklich sind.

Die Aufgabe wird erfindungsgemäß durch Dentalmaterialien gelöst, die
a) 0,1 bis 50 Gew.-% mindestens eines polymerisationsfähigen Phthalsäure-Derivats der allgemeinen Formel I: 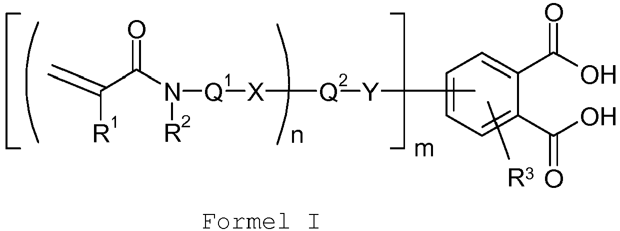 in der
   - R¹: = H oder Methyl, insbesondere H,
   - R²: = H, Methyl oder Ethyl, insbesondere Methyl,
   - Q¹: = ein C₁-C₁₂-Alkylen-Rest, insbesondere ein C₂-C₁₂-Alkylen-Rest, wobei die Kette durch O unterbrochen sein kann, vorzugweise ein linearer C₁-C₆-Alkylen-Rest;
   - Q²: = entfällt oder ein (n+1)-wertiger aliphatischer C₁-C₈-Rest, wobei die Kohlenstoffkette durch O unterbrochen sein kann,
   - X: = entfällt,
   - Y: =O,
   - n, m: = unabhängig voneinander 1 oder 2 sind,
   - R³: = H, CH₃, oder OCH₃,
   wobei die beiden Carboxylgruppen des Benzolrings zusammen eine Anhydridgruppe bilden können,
b) 0,01 bis 10 Gew.-% Initiator für die radikalische Polymerisation,
c) 0 bis 80 Gew.-% eines oder mehrerer zusätzlicher radikalisch polymerisierbarer Monomere (Co-Monomere),
d) 0 bis 30 Gew.-% eines oder mehrerer radikalisch polymerisierbarer, säuregruppenhaltiger Monomere (Haftmonomere),
e) 0 bis 80 Gew.-% Füllstoff und
f) 0 bis 70 Gew.-% Lösungsmittel enthalten.

Q¹ enthält vorzugsweise 0 bis 4 und Q² vorzugsweise 0 bis 6 Heteroatome. Die Formulierung, dass die Kohlenstoffkette durch O oder S unterbrochen ist, ist so zu verstehen, dass die Heteroatome in die Kohlenstoffkette integriert sind, d.h. beidseitig von Kohlenstoffatomen begrenzt werden. Die Anzahl der Kohlenstoffatome ist daher mindestens um 1 größer als die Zahl der Heteroatome.

Besonders bevorzugt sind Verbindungen, in denen alle Variablen eine der bevorzugten Bedeutungen haben.

Es wurde gefunden, dass sich die Phthalsäure-Derivate der Formel (I) nicht nur durch eine hohe Hydrolysebeständigkeit und gute Dentinhaftung auszeichnen, sondern darüber hinaus auch eine geringe Cytotoxizität aufweisen, was für die Anwendung in Dentalwerkstoffen ein erheblicher Vorteil ist.

Die polymerisationsfähigen Phthalsäure-Derivaten der allgemeinen Formel I lassen sich einfach herstellen. Beispielsweise lassen sich OH-funktionalisierte Phthalsäure-Derivate mit Br-Alkyl-funktionalisierten (Meth)acrylamiden zu den Phthalsäure-Derivaten der allgemeinen Formel I umsetzen:

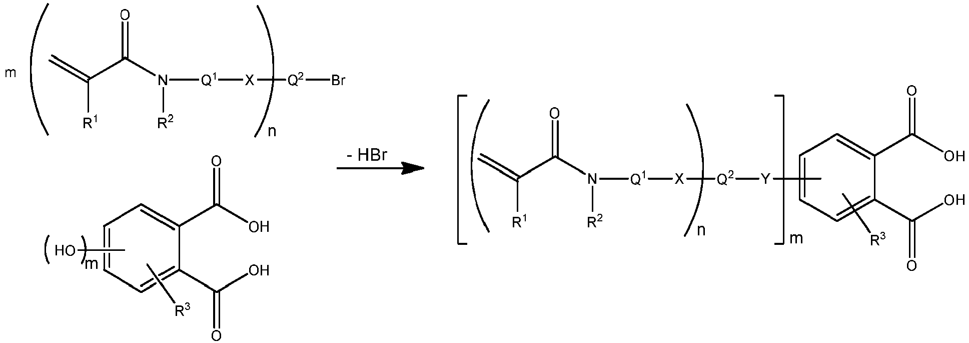

Konkretes Beispiel: Umsetzung von *N*-(11-Bromundecyl)-*N*-methyl-acrylamid mit 4-Hydroxyphthalsäure:

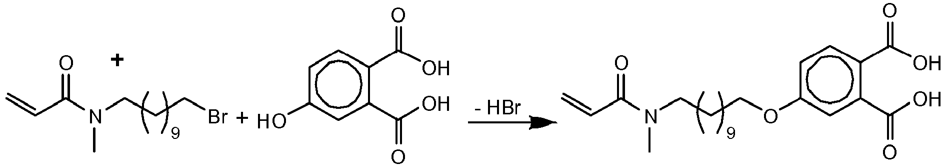

Beispiele für erfindungsgemäß bevorzugte polymerisationsfähige Phthalsäure-Derivate der allgemeinen Formel I sind:

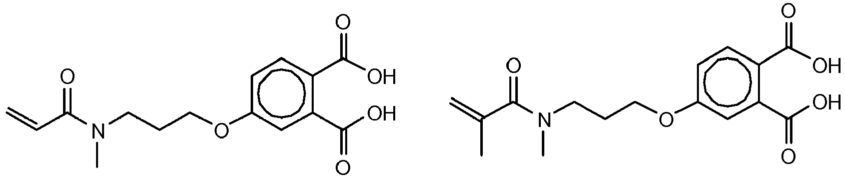

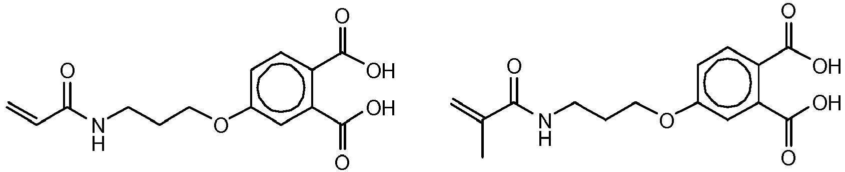

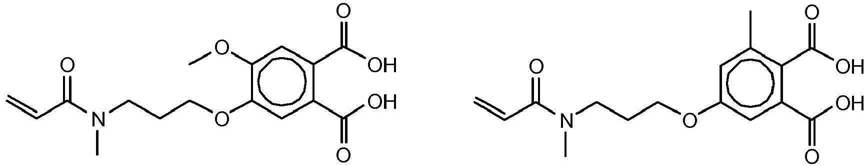

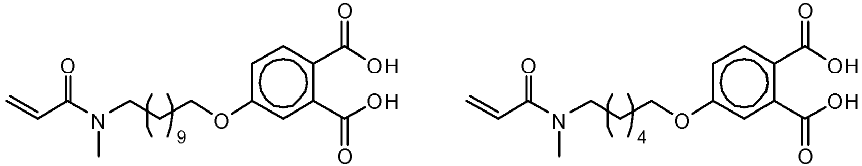

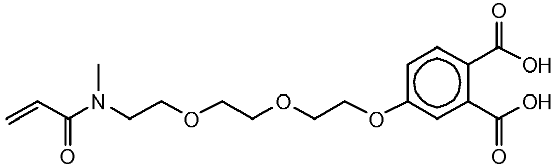

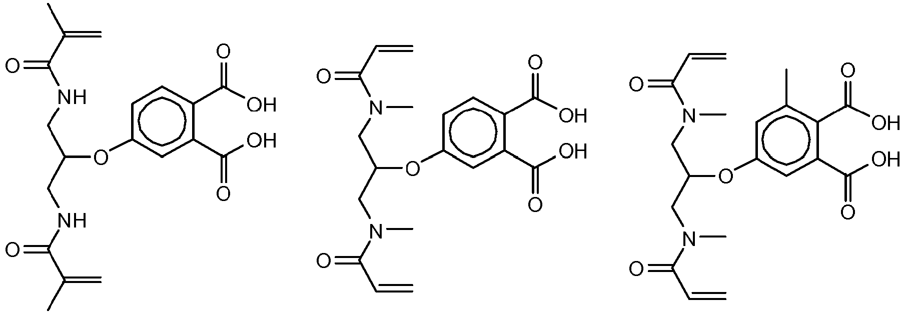

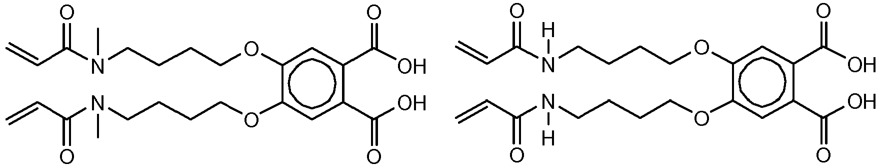

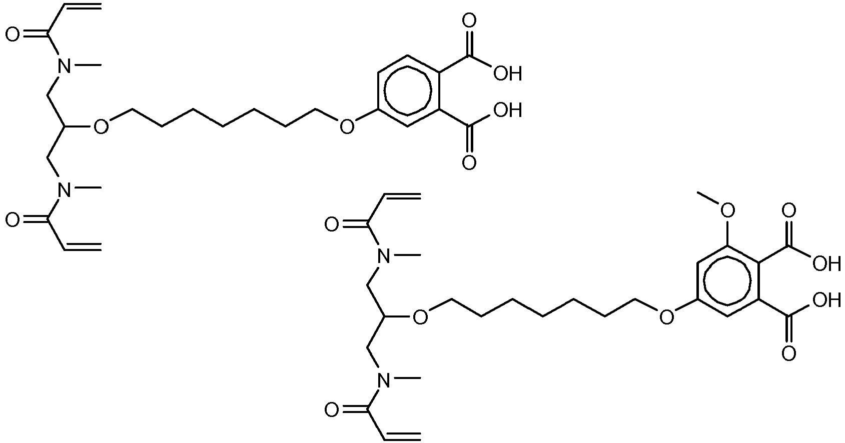

Die erfindungsgemäßen Dentalmaterialien enthalten neben den polymerisationsfähigen Phthalsäure-Derivaten der allgemeinen Formel I vorzugsweise mindestens ein zusätzliches radikalisch polymerisierbares Monomer (Co-Monomer), insbesondere mindestens ein mono- oder polyfunktionelles (Meth)acrylsäurederivat. Unter monofunktionellen (Meth)acrylsäurederivaten werden Verbindungen mit einer, unter polyfunktionellen (Meth)acrylsäurederivaten Verbindungen mit zwei oder mehr, vorzugsweise 2 bis 4 (Meth)acrylsäuregruppen verstanden. Polyfunktionelle Monomere haben eine vernetzende Wirkung.

Erfindungsgemäß bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl-(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Glycerindi(meth)acrylat, 1,4-Butandioldi(meth)acrylat, 1,10-Decandioldi(meth)acrylat oder 1,12-Dodecandioldi(meth)acrylat.

Besonders bevorzugte mono- oder polyfunktionelle (Meth)-acrylsäurederivate sind N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)-acrylamid, N-monosubstituierte Methacrylamide, wie z.B. N-Ethylmethacrylamid oder N-(2-Hydroxyethyl)methacrylamid sowie N-Vinylpyrrolidon oder Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone, wie z.B. 1,6-Bis(3-vinyl-2-pyrrolidonyl)-hexan, oder Bisacrylamide, wie Methylen- oder Ethylenbisacrylamid, Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis-(acryloyl)-piperazin, die durch Umsetzung aus den entsprechenden Diaminen mit (Meth)acrylsäurechlorid synthetisiert werden können.

Vorzugsweise werden Mischungen der voranstehend genannten Monomeren verwendet.

Die erfindungsgemäßen Dentalwerkstoffe können neben Verbindungen der Formel I und ggf. den oben genannten Co-Monomeren vorzugweise auch radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen.

Bevorzugte Monomere mit polymerisationsfähigen Carbonsäuren sind, Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- oder -2,4,6-trimethylphenylester.

Bevorzugte acide polymerisationsfähige Phosphorsäureester sind 2-Methacryloyloxypropylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yldihydrogenphosphat. Bevorzugte polymerisationsfähige Sulfonsäuren sind Vinylsulfonsäure, 4-Vinylphenylsulfonsäure oder 3-(Methacrylamido)-propylsulfonsäure.

Außerdem enthalten die erfindungsgemäßen Dentalwerkstoffe einen Initiator für die radikalische Polymerisation.

Zur Initiierung der radikalischen Photopolymerisation werden vorzugsweise Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil, eingesetzt. Besonders bevorzugt werden Campherchinon und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet. Besonders geeignet sind auch Norrish-Typ-I-Photoinitiatoren, vor allem Acyl- der Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für eine bei Raumtemperatur durchgeführte Polymerisation werden vorzugsweise Redox-Initiatorkombinationen, wie z.B. Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxiden und solchen Reduktionsmitteln, wie z.B. Ascorbinsäure, Barbituraten oder Sulfinsäuren, besonders geeignet.

Weiterhin enthalten die erfindungsgemäß eingesetzten Zusammensetzungen zur Verbesserung der mechanischen Eigenschaften oder zur Einstellung der Viskosität vorzugsweise auch organische oder anorganische Füllstoffpartikel. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂, mit einer mittleren Partikelgröße von 0,005 bis 2,0 µm, vorzugsweise 0,1 bis 1 µm, nanopartikuläre oder mikrofeine Füllstoffe, wie pyrogene Kieselsäure oder Fällungskieselsäure, mit einer mittleren Partikelgröße von 5 bis 200 nm, bevorzugt 10-100 nm, sowie Minifüllstoffe, wie Quarz-, Glaskeramik- oder Glaspulver, mit einer mittleren Teilchengröße von 0,01 bis 1 µm sowie röntgenopake Füllstoffe, wie Ytterbiumtrifluorid oder nanopartikuläres Tantal(V)-oxid bzw. Bariumsulfat, mit einer mittleren Partikelgröße von 10 bis 1000 nm, bevorzugt 10-300 nm. Die Bestimmung der Partikelgröße erfolgt durch Elektronenmikroskopie (TEM und REM) oder durch Streulichtmethoden.

Außerdem können die erfindungsgemäß eingesetzten Zusammensetzungen weitere Additive enthalten, vor allem Lösungsmittel, wie Wasser oder Ethanol bzw. entsprechende Lösungsmittelgemische sowie z.B. Stabilisatoren, Aromastoffe, Farbmittel, mikrobiocide Wirkstoffe, fluoridionenabgebende Additive, optische Aufheller, Weichmacher oder UV-Absorber.

Besonders bevorzugt sind Dentalmaterialien auf der Basis von polymerisationsfähigen Phthalsäure-Derivaten der allgemeinen Formel I; welche die folgenden Bestanteile enthalten:
a) 1 bis 40 Gew.-% und bevorzugt 2 bis 30 Gew.-% polymerisationsfähiges Phthalsäure-Derivat der allgemeinen Formel I,
b) 0,1 bis 3,0 Gew.-% Initiator,
c) 0 bis 60 Gew.-% und bevorzugt 5 bis 50 Gew.-% Co-Monomer,
d) 0 bis 15 Gew.-% Haftmonomer,
e) 0 bis 80 Gew.-% Füllstoff,
f) 0 bis 60 Gew.-% und bevorzugt 0 bis 50 Gew.-% Lösungsmittel.

Die Zusammensetzungen enthalten als Co-Monomer (c) vorzugsweise ein polyfunktionelles Monomer, d.h. ein Monomer mit mehr als einer radikalisch polymerisierbaren Gruppe, oder eine Monomermischung, die überwiegend polyfunktionelle Monomere enthält.

Weiterhin sind solchen Zusammensetzungen bevorzugt, die als Lösungsmittel (f) Wasser enthalten. Die Löslichkeit der Phthalsäure-Derivate der Formel (I) in polaren Lösungsmitteln ist gut.

Der bevorzugte Füllstoffgehalt richtet sich dabei nach der gewünschten Anwendung. Adhäsive enthalten vorzugsweise 0 bis 20 Gew.-% und Zemente und Komposite vorzugsweise 20 bis 80 Gew.-% Füllstoff.

Dies gilt ebenso für den Lösungsmittelgehalt. Adhäsive enthalten vorzugweise 0 bis 60 Gew.-%, besonders bevorzugt 5 bis 60 Gew.-% Lösungsmittel. Dabei sind Adhäsive, die zwischen 0 und 30 Gew.-% und insbesondere zwischen 2 und 20 Gew.-% Wasser enthalten, besonders bevorzugt.

Gegenstand der Erfindung ist auch die Verwendung von Verbindungen der Formel I zur Herstellung von Dentalwerkstoffen, insbesondere von Adhäsiven oder Zementen, ganz besonders selbstätzenden Adhäsiven oder Zementen.

Im folgenden wird die Erfindung anhand von Ausführungsbeispielen näher erläutert.

### Ausführungsbeispiele

### Beispiel 1

### Synthese von 4-[11-(Acryloyl-methyl-amino)-undecyloxy]-phthalsäure (AAUPA)

### 1.Stufe: 4-Hydroxyphthalsäure

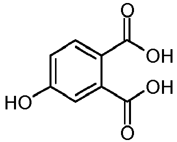

4-Sulfophthalsäure (50% in Wasser, 258.4 g, 0.525 mol) wurde in einem Stahlgefäß portionsweise unter Rühren mit Natriumhydroxid (251.9 g, 6.30 mol) versetzt. Nach Zugabe von ca. einem Drittel an NaOH wurde das zunehmend viskoser werdende Gemisch auf 180 °C erhitzt. Nach beendeter Zugabe wurde das Gemisch weitere 2 h bei 200 °C gerührt. Während des Abkühlens wurde der Rückstand in Wasser (1000 ml) gelöst. Es wurde unter Eiskühlung konz. Salzsäure zugegeben (620 ml, pH = 1). Die Lösung wurde mit Ethylacetat (5x 400 ml) extrahiert. Die vereinigten organischen Phasen wurden über Na₂SO₄ getrocknet, filtriert und am Rotationsverdampfer eingeengt. Der leicht gelbliche Feststoff wurde aus Ethylacetat umkristallisiert. Man erhielt 68.64 g (0.377 mol; 72% Ausbeute) eines weißen Feststoffs.
¹H-NMR (DMSO-*d₆*, 400 MHz) : δ = 6.90 - 6.93 (m, 2H), 7.69 (dd, 1H; *J* = 2.4 Hz, 6.6 Hz), 10.41 (br s, 1H), 12.87 (br s, 2H). ¹³C-NMR (DMSO-*d₆*, 100 MHz): δ = 114.2, 116.2, 120.7, 131.5, 137.3, 160.1, 167.4, 169.5.

### 2. Stufe: 4-Hydroxyphthalsäureanhydrid

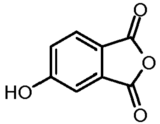

4-Hydroxyphthalsäure (23.80 g, 0.131 mol) wurde im auf 200 °C vorgeheizten Ölbad 1 h erhitzt. Nach dem Abkühlen wurde der bräunliche Feststoff aus Ethylacetat/n-Hexan (1:1) umkristallisiert. Man erhielt 15.82 g (96.4 mmol; 74% Ausbeute) eines weißen Feststoffs.
¹H-NMR (DMSO-*d₆*, 400 MHz): δ = 7.25 (d, 1H; *J* = 2.0 Hz), 7.28 (dd, 1H; *J* = 2.0 Hz, 8.5 Hz), 7.89 (d, 2H, *J* = 8.0 Hz), 11.44 (br s, 1H).
¹³C-NMR (DMSO-*d₆*, 100 MHz): δ = 111.0, 120.7, 123.0, 127.6, 133.9, 162.7, 163.2, 164.8.

### 3. Stufe: 11-Methylamino-undecanol

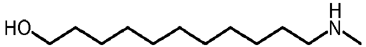

Eine Lösung von 11-Bromundecanol (25.12 g, 0.10 mol) und Methylamin (41% in Wasser, 127 ml, 1.50 mol) in Ethanol (50 ml) wurde 7 h auf 70 °C erhitzt und weitere 16 h bei Umgebungstemperatur gerührt. Das Lösungsmittel wurde abdestilliert. Nach dem Abkühlen wurde der weiße Feststoff in Natronlauge (2N, 200 ml) und Diethylether (300 ml) gelöst. Die Phasen wurden getrennt und die Wasserphase wurde mit Diethylether (2x 100 ml) extrahiert. Die vereinigten organischen Phasen wurden über Na₂SO₄ getrocknet, filtriert und am Rotationsverdampfer eingeengt. Das Rohprodukt wurde aus Diethylether umkristallisiert. Man erhielt 13.23 g (65.7 mmol, 67% Ausbeute) eines weißen Feststoffs (Schmelzpunkt: 58 °C).
¹H-NMR (CDCl₃, 400 MHz) : δ = 1.24 - 1.38 (m, 14H), 1.43 - 1.58 (m, 4H), 2.41 (s, 3H), 2.55 (t, 2H; *J* = 7.4 Hz), 3.57 (t, 2H; *J* = 6.4 Hz).
¹³C-NMR (CDCl₃, 100 MHz): δ = 25.9, 27.3, 29.5, 29.6, 29.6, 29.6, 29.6, 29.8, 33.0, 36.4, 52.1, 62.4.

### 4. Stufe: N-(11-Hydroxy-undecyl)-N-methyl-acrylamid

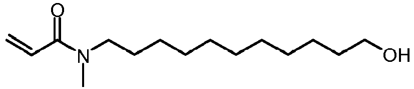

11-Methylaminoundecan-1-ol (10.07 g, 50.0 mmol) wurde in Dichlormethan (30 ml) gelöst. Eine Lösung von Natriumhydroxid (2.40 g, 60.0 mmol) in Wasser (30 ml) wurde zugegeben. Das Gemisch wurde auf -5 °C abgekühlt und eine Lösung von Acrylsäurechlorid (4.75 g, 52.5 mmol) und BHT (10 mg) in Dichlormethan (30 ml) wurde zugetropft. Nach beendeter Zugabe wurde das Reaktionsgemisch 2 h bei -5 °C und weitere 16 h bei RT gerührt. Organische und wässrige Phase wurden getrennt. Die wässrige Phase wurde mit NaCl gesättigt und mit Dichlormethan (2x 50 ml) extrahiert. Die vereinigten organischen Phasen wurden über Na₂SO₄ getrocknet, filtriert und am Rotationsverdampfer eingeengt. Das Rohprodukt wurde mittels Säulenchromatographie gereinigt (SiO₂/Ethylacetat). Man erhielt 10.38 g (40.6 mmol; 81% Ausbeute) einer gelblichen Flüssigkeit.
¹H-NMR (CDCl₃, 400 MHz): δ (2 Isomere) = 1.18 - 1.40 (m, 14H) , 1.50 - 1.62 (m, 4H), 2.99 (s, 1.7H), 3.06 (s, 1.3H), 3.15 (br s, 1H), 3.34 (t, 1.2H; *J* = 7.6 Hz), 3.41 (t, 0.8H; *J* = 7.6 Hz), 3.59 (t, 2H; *J* = 6.7 Hz), 5.66 (dd, 1H; *J* = 2.0 Hz, 10.6 Hz), 6.29 (dq, 1H; *J* = 2.0 Hz, 8.2 Hz), 6.57 (qd, 1H, *J* = 4.4 Hz, 10.4 Hz).
¹³C-NMR (CDCl₃, 100 MHz): δ = 25.8, 26.6, 26.8, 27.1, 28.9, 29.3, 29.4, 29.4, 29.5, 32.7, 34.0, 35.4, 48.1, 50.1, 62.5, 127.6, 127.9, 166.2, 166.5.

### 5. Stufe: N-(11-Bromundecyl)-N-methyl-acrylamid

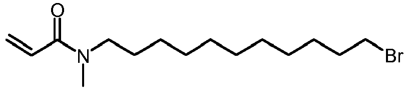

*N*-(11-Hydroxy-undecyl)-*N*-methyl-acrylamid (38.31 g, 0.150 mol) und BHT (10 mg) wurden mit Diethylether (250 ml) versetzt. Phosphortribromid (13.53 g, 50.0 mmol) wurde zugetropft. Der zunächst ungelöste Feststoff löste sich vollständig auf und es schied sich ein gelbliches Öl ab. Das Reaktionsgemisch wurde bei Umgebungstemperatur gerührt. Nach 24 h wurden Wasser (100 ml) und Dichlormethan (200 ml) zugegeben und die Phasen wurden getrennt. Die Wasserphase wurde mit Dichlormethan (2x 100 ml) extrahiert. Die vereinigten organischen Phasen wurden über Na₂SO₄ getrocknet, filtriert, am Rotationsverdampfer eingeengt und am Feinvakuum getrocknet. Das gelbliche Öl wurde in Dichlormethan (100 ml) gelöst und über eine mit Kieselgel gefüllte Fritte filtriert (*n*-Hexan/Ethylacetat 1:1). Der Eluent wurde am Rotationsverdampfer eingeengt und am Feinvakuum getrocknet. Man erhielt 25.25 g (79.3 mmol; 53% Ausbeute) eines gelblichen Öls.
¹H-NMR (DMSO-*d₆*, 400 MHz) : δ (2 Isomere) = 1.18 - 1.56 (m, 16H), 1.74 - 1.83 (m, 2H), 2.86 (s, 1.7H), 3.01 (s, 1.3H), 3.29 - 3.37 (m, 2H), 3.51 4.04 (t, 2H; *J* = 6.8 Hz), 5.61 - 5.65 (m, 1H) , 6.07 - 6.13 (m, 1H) , 6.72 (dd, 1H; *J* = 10.0 Hz, 16.2 Hz) .
¹³C-NMR (DMSO-*d₆*, 100 MHz) : δ = 25.9, 26.3, 26.6, 27.5, 28.1, 28.5, 28.7, 28.8, 28.8, 28.9, 32.2, 33.3, 34.8, 35.0, 46.9, 48.9, 126.6, 128,3, 128.7, 164.9, 165.0.

### 6. Stufe: 4-[11-(Acryloyl-methyl-amino)-undecyloxy]-phthalsäure (AAUPA)

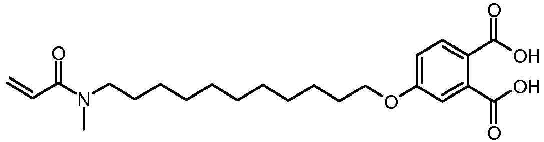

4-Hydroxyphthalsäureanhydrid (6.70 g; 40.8 mmol), BHT (10 mg) und *N*-(11-Bromundecyl)-*N*-methylacrylamid (13.00 g; 40.8 mmol) wurden in *N,N*-Dimethylformamid (100 ml) gelöst. Kaliumcarbonat (5.64 g; 40.8 mmol) wurde zugegeben. Die gelbe Suspension wurde bei RT gerührt. Nach 20 d wurde das Reaktionsgemisch mit Wasser (200 ml) versetzt und 1 h bei RT gerührt. Aus der zunächst trüben Lösung schied sich ein öliger weißer Feststoff ab. Das Lösungsmittel wurde abdekantiert. Der Rückstand wurde in verdünnter wässriger Na₂CO₃-Lösung (5%; 100 ml) gelöst. Die milchig-trübe Wasserphase wurde mit MtBE (5x 100 ml) gewaschen. Die Wasserphase wurde mit verdünnter Salzsäure (2*N*, 100 ml) versetzt (pH = 1) und mit M*t*BE (8x 100 ml) extrahiert. Die vereinigten Extrakte wurden über Na₂SO₄ getrocknet, filtriert und am Rotationsverdampfer eingeengt. Der Rückstand wurde mit Chloroform (150 ml) versetzt und bei RT gerührt. Nach 20 h wurde die Suspension filtriert. Der Filtrationsrückstand wurde mit Chloroform (50 ml) gewaschen und verworfen. Das Filtrat wurde am Rotationsverdampfer eingeengt. Der Rückstand wurde in Chloroform (50 ml) gelöst. Acetonitril (50 ml) wurde zugegeben. Nach 72 h Lagerung bei -18 °C wurde das Lösungsmittel abdekantiert und der Rückstand wurde am Feinvakuum getrocknet. Man erhielt 3.84 g (9.2 mmol; 22% Ausbeute) eines weißen Feststoffs (Schmelzpunkt: 95 °C), der sich z.B. in Ethanol oder Aceton sehr gut löst.
¹H-NMR (DMSO-*d₆*, 400 MHz) : δ (2 Isomere) = 1.15 - 1.35 (m, 12H), 1.36 - 1.50 (m, 4H), 1.68 - 1.75 (m, 2H), 2.86 (s, 1.7H), 3.00 (s, 1.3H), 3.29 - 3.36 (m, 2H), 4.04 (t, 2H; *J =* 6.4 Hz), 5.61 - 5.65 (m, 1H), 6.06 - 6.13 (m, 1H), 6.72 (dd, 1H; *J* = 10.7 Hz, 16.9 Hz), 7.03 - 7.08 (m, 2H), 7.73 (d, 1H; *J* = 8.4 Hz), 12.89 (br, 2H).
¹³C-NMR (DMSO-*d₆*, 100 MHz): δ = 24.3, 24.8, 25.2, 25.5, 27.3, 27.5, 27.6, 27.7, 27.8, 27.9, 32.2, 33.7, 45.8, 47.8, 66.9, 112.3, 114.2, 121.3, 125.6, 127.2, 127.6, 130.1, 136.0, 159.7, 164.0, 166.2, 168.1.

### Beispiel 2

### Radikalische Photopolymerisation des Phthalsäureacrylamides AAUPA aus Beispiel 1

Es wurde eine Mischung aus 2.97 g des Phthalsäureacrylamides AAUPA, 6.95 g des Vernetzers N,N'-Diethyl-1,3-bis(acrylamido)-propan, 0.03 g des Photoinitiators Campher¬chinon und 0.05 g des Aminbeschleunigers 4-(Dimethylamino)-benzoesäure¬ester hergestellt. Ein Tropfen der Mischung wurde auf eine Glasplatte gegeben, mit einer PET-Folie abgedeckt und mit einer Polymerisationslampe (Bluephase; Ivoclar Vivadent AG, Lichtintensität 1000 mW/cm-2) für 20 s bestrahlt: Die bestrahlte Schicht war dann ausgehärtet. Ausserdem wurde die Mischung mittels Photo-DSC (Perkin Elmer DSC 7) untersucht und ergab eine Polymerisationswärme von 301 J/g.

### Beispiel 3

### Untersuchung der Hydrolysestabilität des Phthalsäureacrylamides AAUPA aus Beispiel 1

Es wurde eine Lösung aus 5% des Phthalsäureacrylamides AAUPA stabilisiert mit 200 ppm 2,6-Di-t-butyl-4-methylphenol, 5% Phosphorsäure-d₃, 10% D₂O und 80% DMSO-d₆ hergestellt, diese bei 37 °C gelagert und ¹H-NMR-spektroskopisch untersucht. Nach einer Standzeit von 12 Wochen konnten keine Veränderungen des ¹H-NMR-Spektrums festgestellt werden.

### Beispiel 4

### Herstellung eines lichthärtenden Adhäsivs auf der Basis des Phthalsäureacrylamides AAUPA aus Beispiel 1

Zur Untersuchung der Dentinhaftung auf Rinderzahndentin wurden Adhäsive mit der in Tabelle 1 angegebenen Zusammensetzung hergestellt. Dann wurden Rinderzähne so in Kunststoffzylinder eingebettet, dass sich das Dentin und der Kunststoff in einer Ebene befanden. Nach 15 s Ätzung mit 37 %-iger Phosphorsäure wurde gründlich mit Wasser abgespült. Dann wurde mit einem Microbrush eine Schicht Adhäsiv aufgepinselt, mit einem Luftgebläse zur Entfernung des Lösungsmittels kurz verblasen und für 10 s mit einer Halogenlampe (Astralis 7, Ivoclar Vivadent) belichtet. Auf die Adhäsivschicht wurde ein Kompositzylinder aus einem dentalen Kompositmaterial (Tetric EvoCeram, Ivoclar Vivadent AG) in zwei Schichten von je 1-2 mm aufpolymerisiert. Anschliessend wurden die Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure" zu 31.0 MPa (Adhäsiv A) bzw. 18.2 MPa (Adhäsiv B) bestimmt.

**Tabelle 1: Zusammensetzung der Adhäsive (Angaben in Masse-%)**

| **Komponente** | **Adhäsiv A** | **Adhäsiv B (Vergleich)** |
|---|---|---|
| AAUPA (Bsp. 1) | 10.9 | - |
| 4-MET⁴⁾ | - | 10.9 |
| Glycerindimethacrylat | 9.9 | 9.9 |
| UDMA¹⁾ | 9.9 | 9.9 |
| Bis-GMA²⁾ | 32.7 | 32.7 |
| 2-Hydroxyethylmethacrylat | 14.9 | 14.9 |
| Photoiriitiator³⁾ | 1.7 | 1.7 |
| Ethanol (abs.) | 20.0 | 20.0 |

| | | |
|---|---|---|
| ¹⁾ UDMA ²⁾ Bis-GMA ³⁾ Mischung aus Campherchinon (0.3%), 4-Dimethyl-benzoesäureethylester (0.4%) und dem Acylphosphinoxid Lucerin TPO (1.0%, BASF) ⁴⁾ 4-Methacryloyloxyethyltrimellitsäure | | |

Die Ergebnisse belegen eine sehr gute Dentinhaftung mit dem Adhäsiv auf der Basis des hydrolysestabilen Phthalsäure-Derivates AAUPA.

## Patentansprüche

1. Dentalwerkstoff, **dadurch gekennzeichnet, dass** er
a) 0,1 bis 50 Gew.-% polymerisationsfähiges Phthalsäure-Derivat der allgemeinen Formel I: 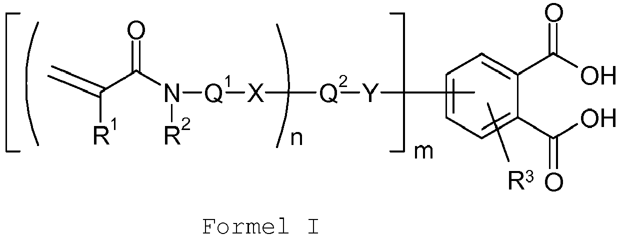 in der
R¹ = H oder Methyl
R² = H oder Methyl,
Q¹ = ein C₁-C₁₂-Alkylen-Rest, wobei die Kette durch O unterbrochen sein kann,
Q² = entfällt oder ein (n+1)-wertiger aliphatischer C₁-C₈-Rest, wobei die Kette durch O unterbrochen sein kann,
X = entfällt,
Y = O,
n, m = unabhängig voneinander 1 oder 2,
R³ = H, CH₃, oder OCH₃,
wobei die beiden Carboxylgruppen des Benzolrings zusammen eine Anhydridgruppe bilden können,
b) 0,01 bis 10 Gew.-% Initiator für die radikalische Polymerisation,
c) 0 bis 80 Gew.-% eines oder mehrerer zusätzlicher radikalisch polymerisierbarer Monomere (Co-Monomere),
d) 0 bis 30 Gew.-% eines oder mehrerer radikalisch polymerisierbarer, säuregruppenhaltiger Monomere (Haftmonomere),
e) 0 bis 80 Gew.-% Füllstoff und
f) 0 bis 70 Gew.-% Lösungsmittel enthält.

2. Dentalwerkstoff nach Anspruch 1, wobei mindestens eine der Variablen eine der folgenden Bedeutungen hat:
R¹ = H,
R² = Methyl,
Q¹ = ein linearer C₁-C₆-Alkylen-Rest;

3. Dentalwerkstoff nach einem der vorhergehenden Ansprüche, der ein oder mehrere zusätzliche radikalisch polymerisierbare Monomere (Co-Monomere) enthält.

4. Dentalwerkstoff nach Anspruch 3, der
Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Glycerindi(meth)acrylat, 1,4-Butandioldi(meth)acrylat, 1,10-Decandioldi(meth)acrylat, 1,12-Dodecandioldi(meth)acrylat, und/oder
ein oder mehrere N-mono- oder -disubstitiuierte Acrylamide, N-Ethylacrylamid, N,N-Dimethacrylamid, N-(2-Hydroxyethyl)acrylamid, N-Methyl-N-(2-hydroxyethyl)acrylamid, ein oder mehrere N-monosubstituierte Methacrylamide, N-Ethylmethacrylamid, N-(2-Hydroxyethyl)methacrylamid, N-Vinylpyrrolidon, ein oder mehrere vernetzende Allylether, und/oder
ein oder mehrere vernetzende Pyrrolidone, 1,6-Bis(3-vinyl-2-pyrrolidonyl)-hexan, ein oder mehrere vernetzende Bisacrylamide, Methylen- oder Ethylenbisacrylamid, ein oder mehrere vernetzende Bis(meth)acrylamide, N,N'-Diethyl-1,3-bis(acrylamido)-propan, 1,3-Bis(methacrylamido)-propan, 1,4-Bis(acrylamido)-butan, 1,4-Bis-(acryloyl)-piperazin,
oder eine Mischung davon enthält.

5. Dentalwerkstoff nach einem der vorhergehenden Ansprüche, der ein oder mehrere radikalisch polymerisierbare, säuregruppenhaltige Monomere (Haftmonomere) enthält.

6. Dentalwerkstoff nach Anspruch 5, der
Maleinsäure, Acrylsäure, Methacrylsäure, 2-(Hydroxymethyl)acrylsäure, 4-(Meth)acryloyloxyethyltrimellitsäure-anhydrid, 10-Methacryloyloxydecylmalonsäure, N-(2-Hydroxy-3-methacryloyloxypropyl)-N-phenylglycin, 4-Vinylbenzoesäure,
und/oder
Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester,
und/oder
2-Methacryloyloxypropylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloylpiperidin-4-yl)-ester, 6-(Methacrylamido)hexyldihydrogenphosphat, 1,3-Bis-(N-acryloyl-N-propyl-amino)-propan-2-yl-dihydrogenphosphat,
und/oder
Vinylsulfonsäure, 4-Vinylphenylsulfonsäure, 3-(Methacrylamido)propylsulfonsäure,
oder eine Mischung davon enthält.

7. Dentalwerkstoff nach einem der vorhergehenden Ansprüche, der organischen und/oder anorganischen Füllstoff enthält.

8. Dentalwerkstoff nach einem der vorhergehenden Ansprüche, der Lösungsmittel, Stabilisatoren, Aromastoffe, Farbmittel, mikrobiocide Wirkstoffe, fluoridionenabgebende Additive, optische Aufheller, Weichmacher und/oder UV-Absorber enthält.

9. Dentalwerkstoff nach einem der vorhergehenden Ansprüche, der
a) 1 bis 40 Gew.-% und bevorzugt 2 bis 30 Gew.-% polymerisationsfähiges Phthalsäure-Derivat der allgemeinen Formel I,
b) 0,1 bis 3,0 Gew.-% Initiator,
c) 0 bis 60 Gew.-% und bevorzugt 5 bis 50 Gew.-% Co-Monomer,
d) 0 bis 15 Gew.-% Haftmonomer,
e) 0 bis 80 Gew.-% Füllstoff,
f) 0 bis 60 Gew.-% und bevorzugt 0 bis 50 Gew.-% Lösungsmittel enthält.

10. Dentalwerkstoff nach Anspruch 9 zur Verwendung als Adhäsiv, der 0 bis 20 Gew.-% Füllstoff enthält.

11. Dentalwerkstoff nach Anspruch 9 zur Verwendung als Komposit, der 20 bis 80 Gew.-% Füllstoff enthält.

12. Verwendung einer Verbindung der Formel I zur Herstellung von Dentalwerkstoff.

13. Verwendung nach Anspruch 12 zur Herstellung eines Adhäsivs oder Zements, oder eines selbstätzenden Adhäsives oder Zements.

## Claims

1. Dental material, comprising
a) 0.1 to 50 wt % polymerisable phthalic acid derivative of the general Formula I: 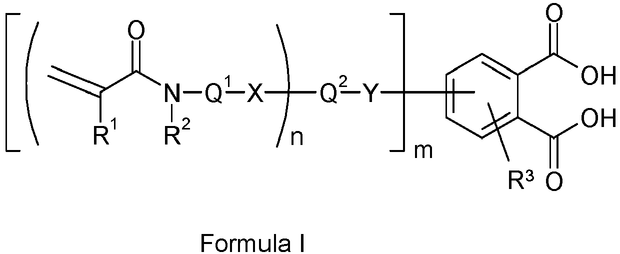 in which
R¹ = H or methyl
R² = H or methyl,
Q¹ = a C₁-C₁₂ alkylene group, wherein the chain can be interrupted by O,
Q² = is absent or is a (n+1)-valent aliphatic C₁-C₈ group, wherein the chain can be interrupted by O,
X = is absent,
Y = O,
n, m = independently of one another are 1 or 2,
R³ = H, CH₃ or OCH₃,
wherein the two carboxyl groups of the benzene ring can together form an anhydride group,
b) 0.01 to 10 wt % initiator for the radical polymerisation,
c) 0 to 80 wt % of one or more additional radically polymerisable monomers (comonomers),
d) 0 to 30 wt % of one or more radically polymerisable, acid group-containing monomers (adherent monomers),
e) 0 to 80 wt % filler and
f) 0 to 70 wt % solvent.

2. Dental material according to claim 1, wherein at least one of the variables has one of the following meanings:
R¹ = H,
R² = methyl,
Q¹ = a linear C₁-C₆ alkylene group;

3. Dental material according to one of the preceding claims which comprises one or more additional radically polymerisable monomers (co-monomers).

4. Dental material according to claim 3 which comprises
methyl, ethyl, hydroxyethyl, butyl, benzyl, tetrahydrofurfuryl or isobornyl (meth)acrylate, bisphenol-A-di(meth)acrylate, bis-GMA (an addition product of methacrylic acid and bisphenol-A-diglycidyl ether), UDMA (an addition product of 2-hydroxyethyl methacrylate (HEMA) and 2,2,4-trimethylhexamethylene diisocyanate), di-, tri- or tetraethylene glycol di(meth)acrylate, trimethylolpropane tri(meth)acrylate, pentaerythritol tetra(meth)acrylate, glycerol di(meth)acrylate, 1,4-butanediol di(meth)acrylate, 1,10-decanediol di(meth)acrylate, 1,12-dodecanediol di(meth)acrylate,
and/or
one or more *N*-mono- or disubstituted acrylamides, *N*-ethylacrylamide, *N,N-*dimethacrylamide, *N*-(2-hydroxyethyl)acrylamide or *N*-methyl-*N*-(2-hydroxyethyl)acrylamide, one or more *N*-monosubstituted methacrylamides, *N-*ethylmethacrylamide, *N*-(2-hydroxyethyl)methacrylamide, *N*-vinylpyrrolidone, one or more cross-linking allyl ethers,
and/or
one or more cross-linking pyrrolidones, 1,6-bis(3-vinyl-2-pyrrolidonyl)-hexane, one or more cross-linking bisacrylamides, methylene or ethylene bisacrylamide, one or more cross-linking bis(meth)acrylamides, *N,N'*-diethyl-1,3-bis(acrylamido)-propane, 1,3-bis(methacrylamido)-propane, 1,4-bis(acrylamido)-butane, 1,4-bis(acryloyl)-piperazine,
or a mixture thereof.

5. Dental material according to one of the preceding claims which comprises one or more radically polymerisable, acid group-containing monomers (adherent monomers).

6. Dental material according to claim 5 which comprises
maleic acid, acrylic acid, methacrylic acid, 2-(hydroxymethyl)acrylic acid, 4-(meth)acryloyloxyethyltrimellitic anhydride, 10-methacryloyloxydecylmalonic acid, *N*-(2-hydroxy-3-methacryloyloxypropyl)-*N*-phenylglycine, 4-vinylbenzoic acid,
and/or
vinylphosphonic acid, 4-vinylphenylphosphonic acid, 4-vinylbenzylphosphonic acid, 2-methacryloyloxyethylphosphonic acid, 2-methacrylamidoethylphosphonic acid, 4-methacrylamido-4-methylpentylphosphonic acid, 2-[4-(dihydroxyphosphoryl)-2-oxa-butyl]-acrylic acid, ethyl or 2,4,6-trimethylphenyl ester of 2-[4-(dihydroxyphosphoryl)-2-oxa-butyl]-acrylic acid,
and/or
2-methacryloyloxypropyl mono- or dihydrogen phosphate, 2-methacryloyloxyethylphenyl hydrogen phosphate, dipentaerythritolpentamethacryloyloxyphosphate, 10-methacryloyloxydecyl dihydrogen phosphate, phosphoric acid mono-(1-acryloyl-piperidine-4-yl) ester, 6-(methacrylamido)hexyl dihydrogen phosphate, 1,3-bis-(*N*-acryloyl-*N-*propylamino)-propane-2-yl dihydrogen phosphate,
and/or
vinylsulfonic acid, 4-vinylphenylsulfonic acid, 3-(methacrylamido)propylsulfonic acid,
or a mixture thereof.

7. Dental material according to one of the preceding claims which comprises organic and/or inorganic filler.

8. Dental material according to one of the preceding claims which comprises solvents, stabilisers, flavourings, dyes, microbiocidal active ingredients, fluoride ion-releasing additives, optical brighteners, plasticisers and/or UV absorbers.

9. Dental material according to one of the preceding claims, which comprises
a) 1 to 40 wt % and preferably 2 to 30 wt % polymerisable phthalic acid derivative of the general Formula I,
b) 0.1 to 3.0 wt % initiator,
c) 0 to 60 wt % and preferably 5 to 50 wt % comonomer,
d) 0 to 15 wt % adherent monomer,
e) 0 to 80 wt % filler,
f) 0 to 60 wt % and preferably 0 to 50 wt % solvent.

10. Dental material according to claim 9 for use as an adhesive which comprises 0 to 20 wt % filler.

11. Dental material according to claim 9 for use as a composite which comprises 20 to 80 wt % filler.

12. Use of a compound of the Formula I for the preparation of dental material.

13. Use according to claim 12 for the preparation of an adhesive or cement or a self-etching adhesive or cement.

## Revendications

1. Matériau dentaire, **caractérisé en ce qu'**il contient
a) 0,1 à 50% en poids de dérivé d'acide phtalique polymérisable de formule générale I : 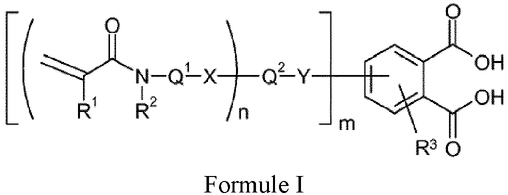 dans laquelle
R¹ = un atome d'hydrogène ou un groupe méthyle
R² = un atome d'hydrogène ou un groupe méthyle,
Q¹ = un radical alkylène en C₁-C₁₂, dont la chaîne peut être interrompue par un atome d'oxygène,
Q² est absent ou représente un radical aliphatique en C₁-C₈ (n+1)-valent, dont la chaîne peut être interrompue par un atome d'oxygène,
X est absent,
Y représente un atome d'oxygène,
n, m valent indépendamment l'un de l'autre 1 ou 2,
R³ = un atome d'hydrogène, un groupe CH₃ ou OCH₃,
les deux groupes carboxy du cycle benzénique pouvant former ensemble un groupe anhydride,
b) 0,01 à 10 % en poids d'amorceur pour la polymérisation radicalaire,
c) 0 à 80 % en poids d'un ou de plusieurs monomère(s) supplémentaire(s) polymérisable(s) par voie radicalaire (comonomère(s)),
d) 0 à 30 % en poids d'un ou de plusieurs monomère(s) contenant des groupes acides, polymérisable(s) par voie radicalaire (monomère(s) adhésif(s)),
e) 0 à 80 % en poids de charge et
f) 0 à 70 % en poids de solvant.

2. Matériau dentaire selon la revendication 1, dans lequel au moins l'une des variables a l'une des significations suivantes :
R¹ = un atome d'hydrogène,
R² = un groupe méthyle,
Q¹ = un radical alkylène en C₁-C₆ linéaire.

3. Matériau dentaire selon l'une quelconque des revendications précédentes, qui contient un ou plusieurs monomère(s) supplémentaire(s) polymérisable(s) par voie radicalaire (comonomère(s)).

4. Matériau dentaire selon la revendication 3, qui contient
du (méth)acrylate de méthyle, d'éthyle, d'hydroxyéthyle, de butyle, de benzyle, de tétrahydrofurfuryle ou d'isobornyle, du di(méth)acrylate de bisphénol A, du bis-GMA (un produit d'addition d'acide méthacrylique et d'éther diglycidylique de bisphénol A), de l'UDMA (un produit d'addition de méthacrylate de 2-hydroxyéthyle (HEMA) et de diisocyanate de 2,2,4-triméthylhexaméthylène), du di(méth)acrylate de di-, tri- ou tétraéthylèneglycol, du tri(méth)acrylate de triméthylolpropane, du tétra(méth)acrylate de pentaérythritol, du di(méth)acrylate de glycérol, du di(méth)acrylate de 1,4-butanediol, du di(méth)acrylate de 1,10-décanediol, du di(méth)acrylate de 1,12-dodécanediol,
et/ou
un ou plusieurs acrylamide(s) mono- ou disubstitué(s) sur l'atome d'azote, N-éthylacrylamide, N,N-diméthacrylamide, N-(2-hydroxyéthyl)acrylamide, N-méthyl-N-(2-hydroxyéthyl)acrylamide, un ou plusieurs méthacrylamide(s) monosubstitué(s) sur l'atome d'azote, N-éthylméthacrylamide, N-(2-hydroxyéthyl)méthacrylamide, N-vinylpyrrolidone, un ou plusieurs éther(s) allylique(s) à action de réticulation,
et/ou
une ou plusieurs pyrrolidone(s) à action de réticulation, 1,6-bis(3-vinyl2-pyrrolidonyl)-hexane, un ou plusieurs bisacrylamide(s) à action de réticulation, méthylène- ou éthylènebisacrylamide, un ou plusieurs bis(méth)acrylamide(s) à action de réticulation, N,N'-diéthyl-1,3-bis(acrylamido)-propane, 1,3-bis(méthacrylamido)-propane, 1,4-bis(acrylamido)-butane, 1,4-bis-(acryloyl)-pipérazine,
ou un mélange de tels composés.

5. Matériau dentaire selon l'une quelconque des revendications précédentes, qui contient un ou plusieurs monomère(s) contenant des groupes acides, polymérisable(s) par voie radicalaire, (monomère(s) adhésif(s)).

6. Matériau dentaire selon la revendication 5, qui contient
de l'acide maléique, acrylique, méthacrylique, 2-(hydroxyméthyl)acrylique, de l'anhydride 4-(méth)acryloyloxyéthyltrimellitique, de l'acide 10-méthacryloyloxydécylmalonique, de la N-(2-hydroxy-3-méthacryloyloxypropyl)-N-phénylglycine, de l'acide 4-vinylbenzoïque,
et/ou
de l'acide vinylphosphonique, 4-vinylphénylphosphonique, 4-vinylbenzylphosphonique, 2-méthacryloyloxyéthylphosphonique, 2-méthacrylamidoéthylphosphonique, 4-méthacrylamido-4-méthyl-pentyl-phosphonique, 2-[4-(dihydroxyphosphoryl)-2-oxa-butyl]-acrylique, 2-[4-(dihydroxyphosphoryl)-2-oxa-butyl]-acrylate d'éthyle ou de 2,4,6-triméthylphényle,
et/ou
du mono- ou dihydrogénophosphate de 2-méthacryloyloxypropyle, de l'hydrogénophosphate de 2-méthacryloyloxyéthylphényle, du pentaméthacryloyloxyphosphate de dipentaérythritol, du dihydrogénophosphate de 10-méthacryloyloxydécyle, du phosphate de mono-(1-acryloylpipéridin-4-yle), du dihydrogénophosphate de 6-(méthacrylamido)hexyle, du dihydrogénophosphate de 1,3-bis-(N-acryloyl-N-propyl-amino)-propan-2-yle,
et/ou
de l'acide vinylsulfonique, 4-vinylphénylsulfonique, 3-(méthacrylamido) propylsulfonique,
ou un mélange de tels composés.

7. Matériau dentaire selon l'une quelconque des revendications précédentes, qui contient une charge organique et/ou une charge inorganique.

8. Matériau dentaire selon l'une quelconque des revendications précédentes, qui contient des solvants, stabilisants, arômes, agents colorants, substances actives microbicides, additifs libérant des ions fluorure, azurants optiques, plastifiants et/ou absorbeurs UV.

9. Matériau dentaire selon l'une quelconque des revendications précédentes, qui contient
a) 1 à 40 % en poids et de préférence 2 à 30 % en poids de dérivé d'acide phtalique polymérisable de formule générale I,
b) 0,01 à 3,0 % en poids d'amorceur,
c) 0 à 60 % en poids et de préférence 5 à 50 % en poids de comonomère,
d) 0 à 15 % en poids de monomère adhésif,
e) 0 à 80 % en poids de charge,
f) 0 à 60 % en poids et de préférence 0 à 50 % en poids de solvant.

10. Matériau dentaire selon la revendication 9, destiné à l'utilisation en tant qu'adhésif, qui contient 0 à 20 % en poids de charge.

11. Matériau dentaire selon la revendication 9, destiné à l'utilisation en tant que composite, qui contient 20 à 80 % en poids de charge.

12. Utilisation d'un composé de formule I pour la production de matériau dentaire.

13. Utilisation selon la revendication 12 pour la production d'un adhésif ou ciment, ou d'un adhésif ou ciment automordançant.
